(19) Europäisches Patentamt
European Patent Office
Office européen des brevets

(11) EP 4 620 941 A2

(12) EUROPEAN PATENT APPLICATION
published in accordance with Art. 153(4) EPC

(43) Date of publication:
**24.09.2025 Bulletin 2025/39**

(21) Application number: **23827309.8**

(22) Date of filing: **02.11.2023**

(51) International Patent Classification (IPC):
***C07C 67/08*** *(2006.01)* ***B01J 3/00*** *(2006.01)*
***C07C 69/54*** *(2006.01)* ***C07B 61/00*** *(2006.01)*

(52) Cooperative Patent Classification (CPC):
**B01D 3/00; C07C 67/08; C07C 69/54;** C07B 61/00

(86) International application number:
**PCT/JP2023/039709**

(87) International publication number:
**WO 2023/249129 (28.12.2023 Gazette 2023/52)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **18.11.2022 JP 2022185001**

(71) Applicant: **Nippon Shokubai Co., Ltd.**
**Osaka-shi, Osaka 541-0043 (JP)**

(72) Inventors:
- **NAKAMURA, Tsukuru**
  **Himeji-shi, Hyogo 671-1282 (JP)**
- **TACHIBANA, Atsushi**
  **Tokyo 100-0011 (JP)**

(74) Representative: **Schön, Christoph**
**Dr. Schön, Neymeyr & Partner mbB**
**Bavariaring 26**
**80336 München (DE)**

# (54) (METH)ACRYLIC ACID ESTER PRODUCTION METHOD

(57) The present invention provides a technique for improving the selectivity of a (meth)acrylic acid ester. The present invention relates to a method of producing a (meth)acrylic acid ester, the method including: reacting (meth)acrylic acid with a secondary alcohol in a reactor in the presence of an acid-type esterification catalyst and a polymerization inhibitor; and supplying an alkene-containing product obtained during production of the (meth)acrylic acid ester to the reactor so that a ratio of an alkene concentration to a secondary alcohol concentration in the reactor is 0.001 or more.

## Description

Technical Field

The present invention relates to a method of producing a (meth)acrylic acid ester. In particular, the present invention relates to a method of producing a (meth)acrylic acid ester by direct esterification between (meth)acrylic acid and a secondary alcohol.

Background Art

It is a known technique to form a (meth)acrylic acid ester by an esterification reaction between an alcohol and (meth)acrylic acid. This reaction is an equilibrium reaction accompanied by generation of water as will be shown below.

[Chem. 1] $CH_2=CH\text{-}COOH + R\text{-}OH \Leftrightarrow CH_2=CH\text{-}COOR + H_2O$ $CH_2=C(CH_3)\text{-}COOH + R\text{-}OH \Leftrightarrow CH_2=C(CH_3)\text{-}COOR + H_2O$

For shifting the equilibrium reaction toward a direction of forming a (meth)acrylic acid ester, it is necessary to remove the water formed during the reaction. On the other hand, this reaction is generally accompanied by a side reaction which forms impurities. These impurities are required to be removed from the viewpoint of providing a (meth)acrylic acid ester of high purity which meets technical requirements for its final use as a monomer for production of a polymer which can be used in many fields of application. Furthermore, for economic reasons, utilizable products present in a crude reaction mixture, in particular, an unreacted reaction material and a catalyst, are preferably recycled in a process as far as possible.

For achieving these objectives, separation/purification processes including a series of combined distillation, extraction and/or static separation are generally carried out, which are difficult to carry out particularly due to the presence of an azeotropic mixture, and are expensive in terms of energy.

For example, the specification of US 6072076 discloses a process for forming an alkyl (meth)acrylate by esterification of (meth)acrylic acid with an alkanol having a chain length in a range of 1 or more and 8 or less carbon atoms in the presence of an acid esterification catalyst.

Also, JP 2014-534972 T (corresponding to the specification of US 2015/0299093) discloses a method for continuous production of 2-octyl acrylate by direct esterification, the method including use of a single reactor and recycling of utilizable compounds, for example, on the one hand an unreacted reaction material and on the other hand an acid catalyst (in particular a sulfur-containing acid-type esterification catalyst, especially a sulfonic acid-type acid catalyst), to continuously produce 2-octyl acrylate of very high purity in a high yield.

Summary of Invention

However, the method disclosed in the specification of US 6072076 cannot be applied in the production of a (meth) acrylic acid ester by an esterification reaction of (meth)acrylic acid with a secondary alcohol. For example, this is because the secondary alcohol is more likely to cause a dehydration reaction that leads to the formation of an alkene and water in the presence of an acid catalyst than a primary alcohol such as 2-ethylhexanol. The water formed is accumulated by reintroducing at least a part of the aqueous phase generated by the esterification reaction into the system, resulting in a risk that the target (meth)acrylic acid ester is more easily decomposed into a secondary alcohol and (meth)acrylic acid by hydrolysis. Furthermore, according to the specification of US 6072076, purification of a crude reaction mixture containing the desired (meth)acrylic acid ester and the residual alcohol is carried out by distillation, in a rectification apparatus, with a long retention time in the presence of an acid catalyst. Synthesis of a (meth)acrylic acid ester using a secondary alcohol involves problems that an alkene and water are generated by the distillation, and that the (meth)acrylic acid ester is decomposed (that is, selectivity of the (meth)acrylic acid ester is low).

In addition, the technique disclosed in JP 2014-534972 T (corresponding to the specification of US 2015/0299093) involves a problem that it is not possible to suppress an intramolecular dehydration reaction of a secondary alcohol in an esterification reaction (that is, selectivity of a (meth)acrylic acid ester is low).

As described above, the techniques disclosed in US 6072076 and JP 2014-534972 T (corresponding to the specification of US 2015/0299093) cannot be said to give a sufficient selectivity of the (meth)acrylic acid ester.

Accordingly, the present invention has been made in view of the above circumstances, and an object thereof is to provide a technique for improving the selectivity of a (meth)acrylic acid ester.

The present inventors have conducted intensive studies to solve the above problems. As a result, they have found that the above problems can be solved by appropriately controlling a ratio of an alkene concentration to a secondary alcohol concentration in a reactor, and thus have completed the present invention.

That is, the above object is achieved by a method of producing a (meth)acrylic acid ester, the method including:

reacting (meth)acrylic acid with a secondary alcohol in a reactor in the presence of an acid-type esterification catalyst and a polymerization inhibitor; and supplying an alkene-containing product obtained during production of the (meth)acrylic acid ester to the reactor so that a ratio of an alkene concentration to a secondary alcohol concentration in the reactor is 0.001 or more.

Brief Description of Drawings

FIG. 1 is a schematic diagram illustrating an embodiment of a (meth)acrylic acid ester production process.
FIG. 2 is a schematic diagram illustrating another embodiment of the (meth)acrylic acid ester production process.
FIG. 3 is a schematic diagram illustrating another embodiment of the (meth)acrylic acid ester production process.
FIG. 4 is a schematic diagram illustrating another embodiment of the (meth)acrylic acid ester production process.

Description of Embodiments

The present invention provides a method of producing a (meth)acrylic acid ester, the method including: reacting (meth)acrylic acid with a secondary alcohol in a reactor in the presence of an acid-type esterification catalyst and a polymerization inhibitor; and supplying an alkene-containing product obtained during production of the (meth)acrylic acid ester to the reactor so that a ratio of an alkene concentration to a secondary alcohol concentration in the reactor is 0.001 or more. According to the present invention, a technique for improving the selectivity of a (meth)acrylic acid ester is provided.
Hereinafter, embodiments of the present invention will be described. It should be noted that the present invention is not only limited to the following embodiments and can be variously modified within the scope of claims. The embodiments described in the present specification can be combined in any way to constitute another embodiment.
In the present specification, the "ratio of the alkene concentration to the secondary alcohol concentration in the reactor" is also simply referred to as "alkene/secondary alcohol concentration ratio" or "alkene/secondary alcohol concentration ratio according to the present invention". In the present specification, the "acid-type esterification catalyst" is also simply referred to as "esterification catalyst" or "esterification catalyst according to the present invention". In the present specification, the term "(meth)acrylic" includes both acrylic and methacrylic. Thus, for example, the term "(meth) acrylic acid" includes both acrylic acid and methacrylic acid.
Physical properties and the like are measured under a condition of room temperature (25 $\pm$ 5°C) unless otherwise specified.
Also, it is to be understood that the terms used herein are used in their ordinary meanings in the art, unless otherwise specified. Accordingly, unless defined otherwise, all the technical terminologies and chemical technical terms used herein have the same meaning as commonly understood by those skilled in the art to which the present invention belongs. In case of conflict, the present specification (including definitions) is prior. Throughout the present specification, it is to be understood that the expression of a singular form includes also a concept of a plural form thereof, unless otherwise specified. Therefore, it is to be understood that an article for a singular form (for example, "a", "an", "the" and the like in the case of English) includes a concept of a plural form thereof, unless otherwise stated.
The present invention is characterized in that an alkene is supplied (recycled) into a reactor so that the alkene is present in the reactor in a specific ratio or higher relative to a secondary alcohol. With this constitution, the selectivity of the (meth)acrylic acid ester can be improved. The detailed mechanism of providing the effect is still unclear, but is considered as follows. The present inventors have found that an intramolecular dehydration reaction of a secondary alcohol occurs in a production process of a (meth)acrylic acid ester to form an alkene, and that this reaction is an equilibrium reaction. For example, when the secondary alcohol is 2-octanol, the following intramolecular dehydration equilibrium reaction occurs.

[Chem. 2] $$C_6H_{13}\text{-}CH(OH)\text{-}CH_3 \Leftrightarrow C_6H_{13}\text{-}CH{=}CH_2 + H_2O$$

The present inventors have surmised that intramolecular dehydration of the secondary alcohol can be suppressed and the equilibrium reaction can be shifted to the secondary alcohol side by recycling the alkene formed in the production process of the (meth)acrylic acid ester to the reactor by circulation in the system. The above assumption was further studied. As a result, surprisingly, a batch reaction is started by charging (supplying and recycling; hereinafter also collectively referred to as "charging") an alkene into a reactor so that, in the batch reaction, the ratio of the alkene concentration to the secondary alcohol concentration (alkene/secondary alcohol concentration ratio) in the reactor at the time of charging (supplying and recycling) is 0.001 or more, whereby the equilibrium reaction can shift in a direction of suppressing the intramolecular dehydration of the secondary alcohol, that is, the reaction can shift to the secondary alcohol side to suppress the formation of the alkene. Therefore, the reaction between the secondary alcohol and the (meth) acrylic acid proceeds more selectively, and the selectivity of the (meth)acrylic acid ester as a final product can be improved.

Also, the yield of the (meth)acrylic acid ester as a final product can also be improved.

It should be noted that the mechanism described above is an assumption and does not limit the technical scope of the present invention.

The ratio of the alkene concentration to the secondary alcohol concentration (alkene/secondary alcohol concentration ratio) in the reactor according to the present invention (at the time of charging) is 0.001 or more. Here, when the alkene/secondary alcohol concentration ratio is less than 0.001, the equilibrium reaction cannot be changed in a direction in which the alkene in the reactor is converted into the secondary alcohol (dehydration of the secondary alcohol occurs), and the selectivity of the (meth)acrylic acid ester decreases. From the viewpoint of the effect of further improving the selectivity of the (meth)acrylic acid ester, the ratio of the alkene concentration to the secondary alcohol concentration (alkene/secondary alcohol concentration ratio) in the reactor (at the time of charging) is preferably 0.002 or more, more preferably 0.004 or more, even more preferably more than 0.004, still more preferably 0.008 or more, even still more preferably more than 0.008, yet even still more preferably 0.080 or more, particularly preferably more than 0.080, and most preferably 0.150 or more. The reaction of the intramolecular dehydration of the secondary alcohol (secondary alcohol ⇄ alkene + water) is an equilibrium reaction, and even if the alkene is charged in an amount more than an amount in which the reaction reaches an equilibrium reaction, the reaction only completely shifts to the secondary alcohol side (the alkene is converted into the secondary alcohol). Therefore, from the viewpoint of the improvement effect of the selectivity of the (meth)acrylic acid ester, an upper limit of the ratio of the alkene concentration to the secondary alcohol concentration (alkene/secondary alcohol concentration ratio) in the reactor (at the time of charging) is not particularly limited. The ratio of the alkene concentration to the secondary alcohol concentration (alkene/secondary alcohol concentration ratio) in the reactor (at the time of charging) is, for example, 1.0 or less, preferably 0.5 or less, more preferably less than 0.300, and even more preferably less than 0.250. When the ratio is equal to or lower than the upper limit, an amount of the liquid to be recovered to the reaction is suppressed, and an amount of a new raw material to be charged per batch is sufficiently secured, so that the productivity of the (meth)acrylic acid ester can be improved.

The alkene concentration in the reactor can be, for example, 100 ppm by mass or more (0.01 mass% or more), 0.10 mass% or more, 0.24 mass% or more, more than 0.24 mass%, 0.48 mass% or more, more than 0.48 mass%, or 0.65 mass% or more. Alternatively, the alkene concentration in the reactor is preferably high from the viewpoint of further improving the selectivity of the (meth)acrylic acid ester. The alkene concentration in the reactor is, for example, 2.0 mass% or more, preferably 2.4 mass% or more, more preferably more than 2.4 mass%, even more preferably 4.4 mass% or more, still more preferably more than 4.4 mass%, even still more preferably 4.5 mass% or more, yet even still more preferably more than 4.5 mass%, yet even still more preferably 7.5 mass% or more, yet even still more preferably more than 7.5 mass%, particularly preferably 8.5 mass% or more, and most preferably more than 8.5 mass%. As described above, the reaction of the intramolecular dehydration of the secondary alcohol (secondary alcohol ⇄ alkene + water) is an equilibrium reaction, and even if the alkene is charged in an amount more than an amount in which the reaction reaches an equilibrium reaction, the reaction only completely shifts to the secondary alcohol side (the alkene is converted into the secondary alcohol). Therefore, from the viewpoint of the improvement effect of the selectivity of the (meth)acrylic acid ester, an upper limit of the alkene concentration in the reactor (at the time of charging) is not particularly limited. The alkene concentration in the reactor is, for example, 40 mass% or less, preferably 30 mass% or less, more preferably 20 mass% or less, and even more preferably 15 mass% or less. When the concentration is equal to or lower than the upper limit, the amount of the liquid to be recovered to the reaction is suppressed, and the amount of the new raw material to be charged per batch is sufficiently secured, so that the productivity of the (meth)acrylic acid ester can be improved. The "alkene concentration in the reactor" means an alkene concentration when the alkene-containing product is charged (supplied and recycled) into the reactor.

The secondary alcohol concentration in the reactor is preferably low from the viewpoint of suppressing the selectivity of the alkene. The secondary alcohol concentration in the reactor is, for example, 30 mass% or more, preferably 35 mass% or more, more preferably 40 mass% or more, even more preferably 45 mass% or more, still more preferably 50 mass% or more, and particularly preferably 55 mass% or more. From the viewpoint of further suppressing the selectivity of the alkene, an upper limit of the secondary alcohol concentration in the reactor is, for example, 80 mass% or less, preferably 75 mass% or less, more preferably 70 mass% or less, even more preferably 65 mass% or less, still more preferably 55 mass% or less, particularly preferably 50 mass% or less, and most preferably less than 50 mass%. The "secondary alcohol concentration in the reactor" means a secondary alcohol concentration when the alkene-containing product is charged (supplied and recycled) into the reactor.

In the present specification, the secondary alcohol concentration and the alkene concentration, and the ratio of the alkene concentration to the secondary alcohol concentration (alkene/secondary alcohol concentration ratio), in the reactor, are measured by the following methods.

Calculation Method of Alkene/Secondary Alcohol Concentration Ratio

As the alkene/secondary alcohol concentration ratio, a value determined by measuring the secondary alcohol concentration and the alkene concentration in the reactor according to the following methods, dividing the obtained alkene

concentration by the secondary alcohol concentration, and rounding off the obtained value to the third decimal place, is adopted. For example, when the alkene/secondary alcohol concentration ratio is calculated as "0.0019 ...", the alkene/-secondary alcohol concentration ratio becomes "0.002" by rounding off the number "9" in the fourth decimal place.

Measurement Method of Secondary Alcohol Concentration and Alkene Concentration in Reactor

The secondary alcohol concentration and alkene concentration were quantitatively analyzed in accordance with the method and conditions for quantification of 2-octanol, 2-octene and 1-methylheptyl acrylate in a reaction liquid described in the following Examples.

Hereinafter, an embodiment of the method of producing a (meth)acrylic acid ester according to the present invention will be described with reference to FIG. 1. In an embodiment of the present invention,

(a) supplying an acid-type esterification catalyst, (meth)acrylic acid, a secondary alcohol, and a polymerization inhibitor (and other components (such as an organic solvent) if used) to a reactor 1 to react (meth)acrylic acid with the secondary alcohol in the presence of the esterification catalyst and the polymerization inhibitor, simultaneously distilling off water formed by the esterification reaction from a column top part of a first distillation column 2 as an azeotropic composition with the secondary alcohol and/or an organic solvent if used, condensing the resulting distillate, and allowing it to stand to separate it into an oil phase and an aqueous phase, while obtaining a reaction mixture A containing a (meth)acrylic acid ester and an alkene in the reactor 1 (step (a));
(b) withdrawing the reaction mixture A from a bottom of the reactor 1, supplying the reaction mixture A to a neutralization and water washing tank 3, and neutralizing and water-washing the reaction mixture A in the neutralization and water washing tank 3, followed by heating to obtain a gas or a condensate liquid thereof, while obtaining a residue (oil phase) in the neutralization and water washing tank 3 as a reaction mixture B (step (b));
(c) supplying the reaction mixture B to a second distillation column 4 and distilling it to separate it into a column bottom liquid of the second distillation column containing the (meth)acrylic acid ester and a recovered alcohol containing the alkene (step (c));
(d) withdrawing the column bottom liquid of the second distillation column 4 from a column bottom part of the second distillation column 4, supplying it to a third distillation column 5, distilling it to separate it into a purified (meth)acrylic acid ester as a final product and a column bottom liquid of the third distillation column 5, and withdrawing the purified (meth) acrylic acid ester from a column top part of the third distillation column 5 (step (d)); and
(e) withdrawing the column bottom liquid of the third distillation column 5 from a column bottom part of the third distillation column 5, supplying it to a treatment apparatus 6, and separating it into a recovered (meth)acrylic acid ester and waste oil (step (e)).

It should be noted that a known method can be used in the same manner or with appropriate modification, except that the alkene is charged (supplied and recycled) into the reactor so that the ratio of the alkene concentration to the secondary alcohol concentration in the reactor (at the time of charging) falls within a specific range, and the present invention is not limited to the following embodiments.

Step (a)

In this step, (meth)acrylic acid, a secondary alcohol, an acid-type esterification catalyst, and a polymerization inhibitor, and, if necessary, an organic solvent (reaction materials) are supplied to the reactor 1 via a pipe 11a. A compound other than these may be further supplied to the reactor 1. In FIG. 1, (meth)acrylic acid, a secondary alcohol, an acid-type esterification catalyst, and a polymerization inhibitor, and, if necessary, an organic solvent are supplied via the same pipe 11a, but may be supplied via another pipe. Particularly, the (meth)acrylic acid may be directly introduced into the reactor via the pipe 11a, and the acid-type esterification catalyst and the polymerization inhibitor may be directly introduced into the reactor via another pipe (not illustrated). A part of the secondary alcohol may be directly introduced into the reactor via a pipe and another part be introduced into the column top part of the first distillation column 2 via another pipe 11c in order to ensure column reflux. Alternatively, as will be described in detail below, the recovered alcohol (secondary alcohol-rich fluid) obtained from the second distillation column 4 (the subsequent purification stage) may be supplied to the reactor 1 via a pipe 11b. Alternatively, as will be described in detail below, the recovered (meth)acrylic acid ester obtained from the treatment apparatus 6 may be supplied to the reactor 1 via a pipe 64 and the pipe 11b.

Here, the acid-type esterification catalysts that can be used are a sulfur-containing acid-type esterification catalyst and an acidic cation exchange resin. Among these, as the sulfur-containing acid-type esterification catalyst, for example, sulfur-containing acid compounds such as sulfuric acid and organic sulfonic acid, for example, sulfuric acid, para-toluenesulfonic acid, methanesulfonic acid, benzenesulfonic acid, dodecylsulfonic acid, and xylenesulfonic acid are used. The acidic cation exchange resin is not limited by resin physical properties such as a resin structure or a degree of

crosslinking, and, for example, both of a porous- or gel-type strongly acidic cation exchange resin and a weakly acidic cation exchange resin can be used, but a porous- or gel-type strongly acidic cation exchange resin is preferably used. Examples of the porous-type strongly acidic cation exchange resin include MSC-1 (available from Dow Inc.), PK-208, PK-212, PK-216, PK-220, and PK-228 (all available from Mitsubishi Chemical Corporation), Amberlyst (trade name)-16, IR-116, IR-118, IR-122, C-26, C-26TR, C-264, and C-265 (all available from Rohm and Haas Company), SPC-108 and SPC-112 (all available from Bayer AG), and KC-470 (available from Sumitomo Chemical Co., Ltd.). Examples of the gel-type strongly acidic cation exchange resin include HCR-S, HCR-W2, and HGR-W2 (all available from Dow Inc.), SK-1B, SK-106, and SK-110 (all available from Mitsubishi Chemical Corporation), Duolite (trade name) C20H and C255LFH (all available from Rohm and Haas Company), and K1221, and K1431 (all available from Bayer AG). These esterification catalysts may be used alone, or two or more thereof may be used in combination. Among these, in terms of handleability and cost, the acid-type esterification catalyst is preferably a sulfur-containing acid-type esterification catalyst, and more preferably sulfuric acid, para-toluenesulfonic acid, or methanesulfonic acid. The sulfur-containing acid-type esterification catalyst preferably includes a sulfur-containing acid compound, more preferably includes at least one compound selected from the group consisting of sulfuric acid and an organic sulfonic acid, even more preferably includes at least one compound selected from the group consisting of sulfuric acid, para-toluenesulfonic acid, methanesulfonic acid, benzenesulfonic acid, dodecylsulfonic acid and xylenesulfonic acid, particularly preferably includes at least one compound selected from the group consisting of sulfuric acid, para-toluenesulfonic acid and methanesulfonic acid, and most preferably includes at least one compound selected from the group consisting of sulfuric acid, para-toluenesulfonic acid and methanesulfonic acid. An amount of the esterification catalyst to be added is, for example, a ratio of 0.5 parts by mass or more and 10 parts by mass or less, and preferably 1 part by mass or more and 5 parts by mass or less relative to 100 parts by mass of (meth)acrylic acid. When two or more esterification catalysts are used in combination, the amount of the esterification catalyst to be added refers to a total amount of these esterification catalysts.

The acid-type esterification catalyst (esterification catalyst, in particular, the sulfur-containing acid-type esterification catalyst), may be supplied to the reactor as it is or supplied to the reactor in the form of a solution (e.g., an aqueous solution). In the latter case, a concentration of the esterification catalyst in a solution may be, for example, about from 50 to 90 mass%, and preferably about from 60 to 80 mass%, but is not limited thereto. When two or more esterification catalysts are used in combination, the concentration of the esterification catalyst refers to a total concentration of these esterification catalysts in the solution.

Acrylic acid or methacrylic acid used as a starting material is produced by a known method, and, for example, is industrially produced from propylene or isobutene. Independently of use of a renewable alcohol, the present invention extends to use of a renewable (meth)acrylic acid during esterification. For example, acrylic acid can also be obtained by a method including a first stage of dehydration of glycerol to obtain acrolein from glycerol, followed by a stage of gas-phase oxidation of acrolein obtained; or by dehydration of 2-hydroxypropionic acid (lactic acid) or 3-hydroxypropionic acid and esters thereof. The (meth)acrylic acid used as a starting material preferably includes at least one compound selected from the group consisting of acrylic acid and methacrylic acid. In an embodiment of the present invention, the (meth)acrylic acid used as a starting material includes acrylic acid. In an embodiment of the present invention, the (meth)acrylic acid used as a starting material is acrylic acid. In an embodiment of the present invention, the (meth)acrylic acid used as a starting material includes methacrylic acid. In an embodiment of the present invention, the (meth)acrylic acid used as a starting material is methacrylic acid. In an embodiment of the present invention, the (meth)acrylic acid used as a starting material is a mixture of acrylic acid and methacrylic acid. An amount of the (meth)acrylic acid to be added is, for example, 10 parts by mass or more and 50 parts by mass or less, preferably 20 parts by mass or more and 35 parts by mass or less relative to 100 parts by mass of a total amount of the reaction materials (a total amount of the (meth)acrylic acid, the secondary alcohol, the acid-type esterification catalyst (especially, sulfur-containing acid-type esterification catalyst) and the polymerization inhibitor, and the organic solvent when added). When the (meth)acrylic acid is added in portions, the amount of the (meth)acrylic acid to be added refers to a total amount of the (meth)acrylic acid. When acrylic acid and methacrylic acid are used in combination, the amount of the (meth)acrylic acid to be added refers to a total amount of acrylic acid and methacrylic acid.

The secondary alcohol is not particularly limited, and examples thereof include isopropanol, 2-pentanol, 3-pentanol, 2-heptanol, 3-heptanol, 2-octanol, 3-octanol, 4-octanol, 2-nonanol, 2-decanol, 2-undecanol, 2-dodecanol, 2-tridecanol, 2-tetradecanol, 2-pentadecanol, 2-hexadecanol, 2-heptadecanol, 2-octadecanol, 2-nonadecanol, 2-eicosanol, and 2-docosanol. These secondary alcohols may be used alone, or two or more thereof may be used in combination. The secondary alcohol preferably includes at least one compound selected from the group consisting of isopropanol, 2-pentanol, 3-pentanol, 2-heptanol, 3-heptanol, 2-octanol, 3-octanol, 4-octanol, 2-nonanol, 2-decanol, 2-undecanol, 2-dodecanol, 2-tridecanol, 2-tetradecanol, 2-pentadecanol, 2-hexadecanol, 2-heptadecanol, 2-octadecanol, 2-nonadecanol, 2-eicosanol, and 2-docosanol, more preferably includes at least one compound selected from the group consisting of 2-heptanol, 3-heptanol, 2-octanol, 3-octanol, 4-octanol, and 2-nonanol, and even more preferably includes 2-octanol. Among them, the secondary alcohol is still more preferably at least one compound selected from the group consisting of 2-octanol, 3-octanol and 4-octanol, and particularly preferably 2-octanol. 2-Octanol is a renewable alcohol, is specifically obtained as a byproduct of sebacic acid obtained by cracking of castor oil, and is useful as bio 1-methylheptyl acrylate (bio

(meth)acrylic acid ester). Thus, in a preferred embodiment of the present invention, the secondary alcohol is 2-octanol (1-methylheptyl (meth)acrylate is produced). 1-Methylheptyl (meth)acrylate has the following structure.

[Chem. 3]

1-METHYLHEPTYL ACRYLATE

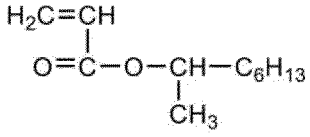

1-METHYLHEPTYL METHACRYLATE

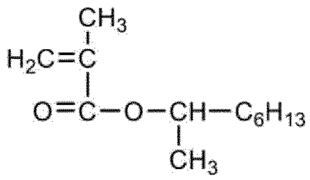

Further, the (meth)acrylic acid ester is obtained by reacting the secondary alcohol with (meth)acrylic acid in equimolar amounts, but the secondary alcohol is preferably used in a larger amount. Specifically, an amount of the secondary alcohol to be added is substantially equal to or more than that of the (meth)acrylic acid, and is, for example, a ratio of 0.9 mol or more and 3.0 mol or less, preferably 1.0 mol or more and 2.0 mol or less per mol of the (meth)acrylic acid. When two or more secondary alcohols are used in combination, the amount of the secondary alcohol to be added refers to a total amount of these secondary alcohols. Similarly, when acrylic acid and methacrylic acid are used in combination, the amount of the (meth)acrylic acid to be added refers to a total amount of acrylic acid and methacrylic acid. As illustrated in FIG. 1, a part of the secondary alcohol is directly introduced into the reactor 1 via the pipe 11a. In addition, a part of the secondary alcohol may be introduced into the column top part of the first distillation column 2 via the pipe 11c. Thus, reflux of the first distillation column 2 can be achieved more reliably.

The polymerization inhibitor is not particularly limited, and examples thereof include phenothiazine, hydroquinone, methoquinone, methylhydroquinone, benzoquinone, hydroquinone monomethyl ether, di(tert-butyl)-p-cresol (BHT), p-phenylenediamine, TEMPO (2,2,6,6-tetramethyl-1-piperazinyloxy), p-tert-butylcatechol, di(tert-butyl)catechol, TEMPO derivatives such as OH-TEMPO, 2,6-tert-butyl-4-methylphenol, and copper (II) dibutyldithiocarbamate. These polymerization inhibitors may be used alone, or two or more thereof may be used in combination. The polymerization inhibitor preferably includes at least one compound selected from the group consisting of phenothiazine, hydroquinone, methoquinone, methylhydroquinone, benzoquinone, hydroquinone monomethyl ether, di(tert-butyl)-p-cresol (BHT), p-phenylenediamine, TEMPO (2,2,6,6-tetramethyl-1-piperazinyloxy), p-tert-butylcatechol, di(tert-butyl)catechol, TEMPO derivatives (e.g., OH-TEMPO), 2,6-tert-butyl-4-methylphenol, and copper (II) dibutyldithiocarbamate, and more preferably includes phenothiazine. Among these, the polymerization inhibitor is preferably phenothiazine. An amount of the polymerization inhibitor to be added is, for example, a ratio of 0.05 parts by mass or more and 5 parts by mass or less, and preferably 0.1 parts by mass or more and 2 parts by mass or less relative to 100 parts by mass of the (meth)acrylic acid. When two or more polymerization inhibitors are used in combination, the amount of the polymerization inhibitor to be added refers to a total amount of these polymerization inhibitors. The polymerization inhibitor may additionally be added in a subsequent purification treatment step. In this case, the amount of the polymerization inhibitor to be added refers to a total amount of the amount thereof initially added to the reactor and the amount thereof additionally added in the subsequent purification treatment step.

The esterification catalyst, (meth)acrylic acid, secondary alcohol and polymerization inhibitor are supplied to the reactor. In this case, water formed by the esterification reaction and/or water separately charged into the reactor and the secondary alcohol form an azeotropic composition. In this case, an amount of the secondary alcohol for forming the azeotropic composition (a total amount of the secondary alcohol to be supplied) is preferably 150 parts by mass or more and 500 parts by mass or less, and more preferably 200 parts by mass or more and 350 parts by mass or less relative to 100 parts by mass of the (meth)acrylic acid. Alternatively, in this case, the amount of the secondary alcohol for forming the azeotropic composition (the total amount of the secondary alcohol to be supplied - the amount of the secondary alcohol to be supplied to the reaction with the (meth)acrylic acid) is preferably 20 parts by mass or more and 100 parts by mass or less, and more preferably 30 parts by mass or more and 80 parts by mass or less relative to 100 parts by mass of the (meth) acrylic acid.

Alternatively, an organic solvent may be further supplied to the reactor in addition to the esterification catalyst, the (meth)acrylic acid, the secondary alcohol, and the polymerization inhibitor. In this case, water formed by the esterification reaction and/or water separately charged into the reactor and the secondary alcohol and/or the organic solvent if used form an azeotropic composition. Examples of the organic solvent that can be used when the organic solvent is further supplied to the reactor include aliphatic hydrocarbons such as hexane, heptane, pentane, and cyclohexane; aromatic hydrocarbons such as toluene and xylene; ethers such as diethyl ether, diisopropyl ether, methyl-tert-butyl ether, and tetrahydrofuran; and ketones such as acetone, methyl ethyl ketone, diisopropyl ketone, and methyl isobutyl ketone. These organic solvents may be used alone, or two or more thereof may be used in combination. The organic solvent preferably includes at least one solvent selected from the group consisting of aliphatic hydrocarbons, aromatic hydrocarbons, ethers, and ketones, more preferably includes an aromatic hydrocarbon, even more preferably includes at least one solvent selected from the group consisting of toluene and xylene, and particularly preferably includes toluene. Among these, the organic solvent is preferably toluene. The organic solvent does not contain any secondary alcohol.

In addition, an amount of the organic solvent to be added is introduced in a ratio of preferably 10 parts by mass or more and 60 parts by mass or less, more preferably more than 10 parts by mass and 45 parts by mass or less, even more preferably 15 parts by mass or more and 40 parts by mass or less, and still more preferably 20 parts by mass or more and 35 parts by mass or less relative to 100 parts by mass of the (meth)acrylic acid. When two or more organic solvents are used in combination, the amount of the organic solvent to be added refers to a total amount of these organic solvents.

The (meth)acrylic acid, secondary alcohol, esterification catalyst, and polymerization inhibitor and, if necessary, organic solvent are supplied to the reactor, and then the (meth)acrylic acid and the secondary alcohol are subjected to a batch reaction in the reactor in the presence of the esterification catalyst. Here, conditions for the batch reaction are not particularly limited, and the same conditions as known ones can be applied. For example, a reaction temperature is preferably 40°C or higher and 120°C or lower, and more preferably 50°C or higher and 110°C or lower. In particular, when the reaction temperature is within the above range, the formation of impurities such as an alkene can be effectively suppressed. In addition, sufficient productivity can be achieved while a reaction rate is secured. A reaction time is preferably 4 hours or more and 24 hours or less, and more preferably 5 hours or more and 15 hours or less, as the reaction time after a predetermined reaction temperature is reached. A pressure at the time of the reaction is not particularly limited and can be appropriately selected from under normal pressure, under reduced pressure, or under increased pressure according to the reaction form. The pressure is preferably under normal pressure or under reduced pressure, more preferably under reduced pressure, and particularly preferably adjusted (reduced) so that the reaction temperature is reached. After the reaction for a predetermined time, the reaction can be terminated by cooling the reactor.

The reactor 1 may be provided with an external heating means (for example, a heat exchanger). In this case, the reactor 1 is heated to a predetermined temperature by the heating means. The reactor 1 may be provided with a stirrer. The esterification reaction is an equilibrium reaction accompanied by generation of water as will be shown below. Therefore, in order to shift the reaction toward the ester formation side, it is necessary to remove the formed water out of the system.

[Chem. 4] $CH_2=CH\text{-}COOH + R\text{-}OH \Leftrightarrow CH_2=CH\text{-}COOR + H_2O$ $CH_2=C(CH_3)\text{-}COOH + R\text{-}OH \Leftrightarrow CH_2=C(CH_3)\text{-}COOR + H_2O$

Therefore, the reactor 1 is provided with a distillation column (first distillation column) 2. As a result, the formed water can be distilled off out of the system to remove water from the reaction system. Here, the first distillation column 2 can be, for example, a packed distillation column or plate distillation column having a theoretical plate number of 5 or more and 15 or less (for example, about 10). The reactor 1 and the first distillation column 2 are shown as separate facilities in FIG. 1, but may be integrated into one facility.

As a distillation method in the first distillation column 2, a known method such as simple distillation (for example, flash distillation) or molecular distillation (thin film distillation) can be used, but the method is not particularly limited thereto. At this time, the simple distillation is intended to be batch distillation without a rectifying portion and can be carried out by a general apparatus. The molecular distillation (thin film distillation) can be carried out using a Hickman type distiller, a falling-film type distiller, a rotor tray type distiller, a brush type molecular distillation apparatus, or the like. The first distillation column 2 may be heated by a heating means such as a thermosiphon or a forced circulation external heat exchanger. In this case, the first distillation column 2 is heated to a predetermined temperature by the heating means. When the reactor 1 and the first distillation column 2 are provided with an external heating means, the heating means of the reactor 1 and the heating means of the first distillation column 2 may be installed separately or may be the same (shared). Conditions for the distillation are not particularly limited. A distillation pressure is performed under a reduced pressure of, for example, 30 Torr or more and 650 Torr or less (1 Torr = about 1.3 hPa) or under atmospheric pressure, but is not limited thereto. A distillation temperature (particularly, column bottom temperature) is, for example, 40°C or higher and 110°C or lower, preferably 50°C or higher and 100°C or lower, but is not limited thereto. A distillation time is, for example, 4 hours or more and 24 hours or less, preferably 5 hours or more and 15 hours or less, or the like, but is not limited thereto. Under such conditions, the formed water can be efficiently distilled out of the system.

The water formed by the esterification reaction forms an azeotropic composition with the secondary alcohol and/or the organic solvent if used and is distilled off as a gas from the column top part of the first distillation column 2. This gas is condensed into a liquid in a condenser (not illustrated), charged into an intermediate tank (not illustrated), allowed to stand, and thus separated into an oil phase and an aqueous phase (oil-water separation). A part or all of the oil phase may be charged into the first distillation column 2 as reflux (not illustrated). Specifically, the oil phase contains the (meth)acrylic acid ester formed by the esterification reaction, the alkene, a small amount of (meth)acrylic acid, the secondary alcohol, and (the organic solvent if used). Therefore, a part of the oil phase may be supplied (recycled) to the reactor 1 via a pipe 22a and the pipe 11b to adjust the alkene/secondary alcohol concentration ratio in the reactor 1 (at the time of charging) to 0.001 or more (or the preferable range described above). The supply (recycling) of the oil phase is generally carried out in batches. That is, in an embodiment of the present invention, the alkene-containing product obtained during the production of the (meth)acrylic acid ester is (i) an oil phase obtained by supplying an azeotropic composition of water formed by the reaction and/or water charged into the reactor and the secondary alcohol to a first distillation column, distilling the azeotropic composition, and condensing a gas distilled off from a column top part of the first distillation column. In an embodiment of the present invention, the alkene-containing product obtained during the production of the (meth)acrylic acid ester is (ii) an oil phase obtained by further carrying out the reaction in the presence of an organic solvent (excluding a secondary alcohol), supplying an azeotropic composition of water formed by the reaction and/or water charged into the reactor and the secondary alcohol and/or the organic solvent to a first distillation column, distilling the azeotropic composition, and condensing a gas distilled off from a column top part of the first distillation column.

As described above, during the esterification reaction, a mixture containing mainly an azeotropic composition of the secondary alcohol and/or the organic solvent if used and water and a small amount of (meth)acrylic acid is distilled, and the azeotropic composition is condensed, and then separated into two phases, an oil phase and an aqueous phase. To this end, the reactor 1 and the first distillation column (for example, packed distillation column) 2 may be provided with a condenser supplied with water (for example, water at 25°C) and a decanter receiving the condensed azeotropic composition. This decanter may be a decanter equipped with a system which makes it possible to control a lower aqueous phase to a constant position by automatically removing the reaction water formed by opening a solenoid valve. It may be operated under reduced pressure by being adjusted with a vacuum system.

In addition, the aqueous phase part may be partially discarded, or may be partially or entirely supplied to the neutralization and water washing tank 3 via a pipe 22b. The aqueous phase part is not reintroduced into the first distillation column 2. As a result, the equilibrium of the esterification reaction can be constantly shifted toward the (meth)acrylic acid ester formation side (i.e., selectivity and yield can be improved). Alternatively, the secondary alcohol and the (meth)acrylic acid contained at a low concentration may be recovered by subjecting the aqueous phase part to a biological treatment and subjecting the aqueous phase part to a distillation treatment before discharge.

By the esterification reaction, a reaction mixture (reaction mixture A) containing a (meth)acrylic acid ester and an alkene is formed in the reactor 1. The reaction mixture A may generally contain the unreacted secondary alcohol, the unreacted (meth)acrylic acid, the esterification catalyst, the polymerization inhibitor, other high-boiling-point byproducts, and the like in addition to the (meth)acrylic acid ester and the alkene. The (meth)acrylic acid ester is generally contained in the reaction mixture A in a proportion of about 50 mass% or more and 95 mass% or less.

Step (b)

In this step, the reaction mixture A obtained in the above step (a) is withdrawn from the bottom of the reactor 1 and supplied to the neutralization and water washing tank 3 via a pipe 31. In addition, the aqueous phase separated from the first distillation column 2 is supplied to the neutralization and water washing tank 3 via the pipe 22b. The reaction mixture A is neutralized and washed with water in the neutralization and water washing tank 3 by supplying the aqueous phase and, if necessary, additional water. Thus, an acid component and an alkali component in the reaction mixture A are removed. Here, the neutralization and water washing tank 3 may be provided with a stirring device, and may be a tank in which retention is performed while stirring (stirring retention tank) or a mixer.

Neutralization is carried out with a base. Thus, the acid component in the reaction mixture A is neutralized. As the base, for example, sodium hydroxide, potassium hydroxide, sodium carbonate, ammonium carbonate and the like are used. These bases may be used alone, or two or more thereof may be used in combination. The base preferably includes at least one compound selected from the group consisting of sodium hydroxide, potassium hydroxide, sodium carbonate, and ammonium carbonate, and more preferably includes sodium hydroxide. Among these, the base is preferably sodium hydroxide. The base may also be used in the form of an aqueous solution. An amount of the base to be added has only to be an amount that enables neutralization of the acid component in the reaction mixture A, and can be appropriately selected in consideration of the amount of the esterification catalyst to be supplied to the reactor, and the like.

After the neutralization (when stirring is performed, the stirring is stopped), the obtained mixture is allowed to stand, and thus separated into an oil phase and an aqueous phase (oil-water separation). The aqueous phase part (the acid component containing the esterification catalyst and the alkali component (base)) is removed out of the system via a

pipe 33, and only the oil phase is left in the neutralization and water washing tank 3 (neutralization step).

Next, water is charged into the neutralization and water washing tank 3, and, if necessary, stirred to remove the base remaining in the oil phase (water washing). After the water washing (when stirring is performed, the stirring is stopped), the obtained mixture is allowed to stand, and thus separated into an oil phase and an aqueous phase (oil-water separation). Among them, the aqueous phase part (alkali component (base)) is removed out of the system via the pipe 33, and only the oil phase is left in the neutralization and water washing tank 3 (water washing step).

The neutralization step and the water washing step may be performed as separate steps in a batch operation, or neutralization and water washing may be carried out continuously in a continuous extraction column. In addition, each of the neutralization step and the water washing step may be performed once or repeatedly.

By the steps, most or all of the esterification catalyst contained in the reaction mixture A is removed out of the system as an aqueous phase.

On the other hand, the oil phase after water washing is heated. This allows the secondary alcohol and the alkene, and (the organic solvent if used) to be recovered as a gas, which is condensed if necessary. The gas or condensate liquid contains the alkene formed by the esterification reaction. Therefore, the gas or condensate liquid may be supplied (recycled) to the reactor 1 via a pipe 32 and the pipe 11b to adjust the alkene/secondary alcohol concentration ratio in the reactor 1 (at the time of charging) to 0.001 or more (or the preferable range described above). That is, in an embodiment of the present invention, the alkene-containing product obtained during the production of the (meth)acrylic acid ester is (iii) a gas or a condensate liquid of the gas, obtained after supplying a reaction mixture A obtained in the reaction to a neutralization and water washing tank, and neutralizing and water-washing the reaction mixture A. In an embodiment of the present invention, the alkene-containing product obtained during the production of the (meth)acrylic acid ester is (iii') a gas or a condensate liquid of the gas, obtained after supplying a reaction mixture A obtained in the reaction to a neutralization and water washing tank, neutralizing and water-washing the reaction mixture A, separating the reaction mixture A into an aqueous phase and an oil phase, and then heating the oil phase. The supply (recycling) of the gas or condensate liquid is generally carried out in batches.

A method for heating the oil phase after water washing is not particularly limited, and the oil phase may be heated with a heater or distilled. Preferably, the oil phase after water washing is distilled. As a distillation method when distilling the oil phase after water washing, a known method such as simple distillation (for example, flash distillation) or molecular distillation (thin film distillation) can be used, but the method is not particularly limited thereto. For example, a distillation column such as a packed column or plate column having a theoretical plate number of 5 or more and 15 or less can be used. The distillation column may be heated by a heater such as a thermosiphon or a forced circulation external heat exchanger. Here, conditions for the distillation are not particularly limited. A distillation pressure is performed under a reduced pressure of, for example, 20 Torr or more and 200 Torr or less (1 Torr = about 1.3 hPa) or under atmospheric pressure, but is not limited thereto. A distillation temperature (particularly, column bottom temperature) is 50°C or higher and 150°C or lower, preferably 70°C or higher and 120°C or lower, or the like, but is not limited thereto. A distillation time is 4 hours or more and 24 hours or less, preferably 5 hours or more and 15 hours or less, or the like, but is not limited thereto. Under such conditions, the gas (for example, secondary alcohol and alkene) can be efficiently distilled off out of the system.

After the treatment, the oil phase (reaction mixture B) containing the (meth)acrylic acid ester remains in the neutralization and water washing tank 3.

In the present embodiment, as illustrated in FIG. 1, the step (a) and the step (b) are carried out by different apparatuses. Alternatively, the steps (a) and (b) are carried out in batches. Therefore, as illustrated in FIG. 2, the steps (a) and (b) may be carried out in one apparatus (another embodiment (I)). That is, in the other embodiment (I), the reactor 1 also functions as the neutralization and water washing tank 3 ("1 (3)" in FIG. 2). Specifically, in the other embodiment (I), after completion of the reaction in the above step (a), the aqueous phase or water separated from the first distillation column 2 is supplied to the reaction mixture A in a reactor 1 (3) obtained in the above step (a) via the pipe 22b. In the other embodiment (I), the steps (a) and (b) are carried out in the same manner as the steps (a) and (b), except that the reactor 1 and the neutralization and water washing tank 3 are integrated (the reactor 1 (3) is used). In the case of the other embodiment (I), a tank 25 separately storing the aqueous phase separated from the first distillation column 2 until the aqueous phase is supplied to the reactor 1 (3) may be provided. Alternatively, as illustrated in FIGS. 3 and 4, the aqueous phase separated from the first distillation column 2 may be removed (discharged) via a pipe 22c. In this case, water is separately supplied to carry out the step (b) (neutralization step and water washing step). In FIGS. 3 and 4, the entire amount of the aqueous phase separated from the first distillation column 2 is removed via the pipe 22c, but a part of the aqueous phase may be removed via the pipe 22c and the remainder may be supplied to the neutralization and water washing tank 3 or the reactor 1 (3) (not illustrated). As illustrated in FIGS. 2 and 4, a part or all of the oil phase obtained by condensation and/or oil-water separation of the gas distilled off from the column top part of the first distillation column 2 may be supplied (recycled) to the reactor 1 (3) via the pipes 22a (32) and 11b so as to achieve a desired alkene/secondary alcohol concentration ratio.

In the above step (b), an oil phase (reaction mixture B) is obtained in neutralization and water washing tank 3 (FIGS. 1 and 3) or the reactor 1 (3) (FIGS. 2 and 4).

Step (c)

In this step, the oil phase (reaction mixture B) obtained in the above step (b) is supplied to the second distillation column 4 via a pipe 41 and distilled. As a result, it is separated into a column bottom liquid of the second distillation column containing the (meth)acrylic acid ester and a recovered alcohol containing the alkene and the secondary alcohol (secondary alcohol-rich fluid) (light-boiling impurity). As described above, the steps (a) and (b) are generally carried out in batches, but the steps subsequent to these steps are carried out continuously (continuous operation).

In the second distillation column 4, the oil phase (reaction mixture B) obtained in the neutralization and water washing tank 3 or reactor 1 (3) is distilled. The gas distilled off from the column top or the condensate thereof contains the alkene formed by the esterification reaction, the secondary alcohol, the (meth)acrylic acid and a small amount of the (meth) acrylic acid ester (light-boiling impurity). Therefore, the gas or condensate liquid may be supplied (recycled) to the reactor 1 via a pipe 42 and the pipe 11b to adjust the alkene/secondary alcohol concentration ratio in the reactor 1 (at the time of charging) to 0.001 or more (or the preferable range described above). That is, in an embodiment of the present invention, the alkene-containing product obtained during the production of the (meth)acrylic acid ester is (iv) a gas or a condensate liquid of the gas, distilled off from a column top part of a second distillation column, after supplying a reaction mixture A obtained in the reaction to a neutralization and water washing tank, neutralizing and water-washing the reaction mixture A, then supplying an obtained reaction mixture B to the second distillation column, and distilling the reaction mixture B. In an embodiment of the present invention, the alkene-containing product obtained during the production of the (meth)acrylic acid ester is (iv') a gas or a condensate liquid of the gas, distilled off from a column top part of a second distillation column, after supplying a reaction mixture A obtained in the reaction to a neutralization and water washing tank, neutralizing and water-washing the reaction mixture A, separating the reaction mixture A into an aqueous phase and an oil phase (reaction mixture B), then supplying the oil phase (reaction mixture B) to the second distillation column, and distilling the oil phase. The supply (recycling) of the gas or condensate liquid (recovered alcohol) is generally carried out in batches.

In an embodiment of the present invention, the alkene-containing product obtained during the production of the (meth)acrylic acid ester is at least one selected from the following (i) to (iv):

(i) an oil phase obtained by supplying an azeotropic composition of water formed by the reaction and/or water charged into the reactor and the secondary alcohol to a first distillation column, distilling the azeotropic composition, and condensing a gas distilled off from a column top part of the first distillation column;

(ii) an oil phase obtained by further carrying out the reaction in the presence of an organic solvent (excluding a secondary alcohol), supplying an azeotropic composition of water formed by the reaction and/or water charged into the reactor and the secondary alcohol and/or the organic solvent to a first distillation column, distilling the azeotropic composition, and condensing a gas distilled off from a column top part of the first distillation column;

(iii) a gas or a condensate liquid of the gas, obtained after supplying a reaction mixture A obtained in the reaction to a neutralization and water washing tank, and neutralizing and water-washing the reaction mixture A; and

(iv) a gas or a condensate liquid of the gas, distilled off from a column top part of a second distillation column, after supplying a reaction mixture A obtained in the reaction to a neutralization and water washing tank, neutralizing and water-washing the reaction mixture A, then supplying an obtained reaction mixture B to the second distillation column, and distilling the reaction mixture B.

Here, as a distillation method in the second distillation column 4, a known method such as simple distillation (for example, flash distillation) or molecular distillation (thin film distillation) can be used, but the method is not particularly limited thereto. As the second distillation column 4, a packed column or a plate column having a theoretical plate number of 5 or more and 20 or less (for example, theoretical plate number = about 15) can be used. The second distillation column 4 may be heated by a heater such as a thermosiphon or a forced circulation external heat exchanger. Here, conditions for the distillation are not particularly limited. A distillation pressure is performed under a reduced pressure of, for example, 5 Torr or more and 50 Torr or less (1 Torr = about 1.3 hPa) or under atmospheric pressure, but is not limited thereto. A distillation temperature (particularly, column bottom temperature) is, for example, 80°C or higher and 150°C or lower, preferably 100°C or higher and 130°C or lower, but is not limited thereto. Under such conditions, components having a relatively low boiling point (light-boiling impurity containing the secondary alcohol as a main component) can be efficiently distilled off out of the system. If necessary, the polymerization inhibitor may be introduced from the column top part of the second distillation column 4. In this case, the polymerization inhibitor may be used in the form of a solution in which it is dissolved in a secondary alcohol.

A fluid containing a large amount of the (meth)acrylic acid ester (column bottom liquid) remains at the column bottom part of the second distillation column 4 (reaction mixture C).

The operation after the second distillation column 4 is continuous, but, in this case, the recycling (return) of the gas or the condensate thereof (recovered alcohol) is also a batch operation because a batch operation is performed in the return destination. On the other hand, the column bottom liquid of the second distillation column 4 containing the (meth)acrylic acid ester is sent to the third distillation column 5 via a pipe 51.

Step (d)

In this step, the column bottom liquid (reaction mixture C) of the second distillation column 4 obtained in the above step (c) is withdrawn from the column bottom part of the second distillation column 4, supplied to the third distillation column 5 via the pipe 51, and distilled. As a result, the purified (meth)acrylic acid ester as a final product is distilled off from the column top of the distillation column 5 via a pipe 52, and a column bottom liquid (column bottom liquid D) remains in the column bottom part of the third distillation column 5. The purified (meth)acrylic acid ester is usually shipped as a product. On the other hand, the column bottom liquid (column bottom liquid D) of the third distillation column 5 contains high-boiling-point impurities (for example, a Michael adduct generated by addition of a secondary alcohol to a (meth)acrylic acid ester and an ester of a (meth)acrylic acid dimer) and a polymerization inhibitor, and is fed to the next step (treatment apparatus 6) via a pipe 61.

Here, as a distillation method in the third distillation column 5, a known method such as simple distillation (for example, flash distillation) or molecular distillation (thin film distillation) can be used, but the method is not particularly limited thereto. For example, a packed column and a plate column having a theoretical plate number of 5 or more and 20 or less (for example, theoretical plate number = about 5 or more and 10 or less) can be used. The third distillation column 5 may be heated by a heater such as a thermosiphon or a forced circulation external heat exchanger. Here, conditions for the distillation are not particularly limited. A distillation pressure is performed under a reduced pressure of, for example, 5 Torr or more and 50 Torr or less (1 Torr = about 1.3 hPa) or under atmospheric pressure, but is not limited thereto. A distillation temperature (particularly, column bottom temperature) is, for example, 80°C or higher and 150°C or lower, preferably 100°C or higher and 130°C or lower, but is not limited thereto. Under such conditions, the purified (meth)acrylic acid ester can be efficiently distilled off out of the system. If necessary, the polymerization inhibitor may be introduced from the column top part of the third distillation column 5. In this case, the polymerization inhibitor may be used in the form of a solution in which it is dissolved in a (meth)acrylic acid ester.

In the present embodiment, the steps (c) and (d) are performed in different apparatuses as illustrated in FIG. 1, but may be performed in one apparatus as illustrated in FIG. 3 (another embodiment (II)). That is, in the other embodiment (II), the second distillation column 4 and the third distillation column 5 are used as the same apparatus ("4 (5)" in FIG. 3). In the other embodiment (II), the steps (c) and (d) are carried out under the same conditions as those described for the steps (c) and (d) except that they are carried out in one distillation column. Here, when the steps (c) and (d) are carried out in a batch operation, the oil phase (reaction mixture B) obtained in the above step (b) is supplied to the second distillation column 4 (5) via the pipe 41 and distilled (first distillation), and then the column bottom liquid (reaction mixture C) remaining at the bottom is distilled in the second distillation column 4 (5) (second distillation). The gas or condensate liquid (light-boiling impurity) distilled off from the top of the second distillation column 4 (5) in the step (c) (first distillation) is supplied (recycled) to the reactor 1 via the pipes 42 and 11b. At this time, it may be supplied (recycled) so as to achieve a desired alkene/secondary alcohol concentration ratio. Next, in the step (d) (second distillation), the purified (meth)acrylic acid ester as the final product is distilled off from the top of the second distillation column 4 (5) via a pipe 52a by switching a distillation line. Finally, the column bottom liquid (column bottom liquid D) remains at the column bottom part of the second distillation column 4 (5). When the steps (c) and (d) are carried out continuously, the gas or condensate liquid (light-boiling impurity) distilled off from the top of the second distillation column 4 (5) in the step (c) is supplied (recycled) to the reactor 1 via the pipes 42 and 11b, and the purified (meth)acrylic acid ester as the final product is distilled off from the side of the second distillation column 4 (5) via a pipe 52b (side cutting). At this time, it may be supplied (recycled) so as to achieve a desired alkene/secondary alcohol concentration ratio. Finally, the column bottom liquid (column bottom liquid D) remains at the column bottom part of the second distillation column 4 (5).

The embodiments described in the present specification can be combined in any way to constitute another embodiment. For example, as illustrated in FIG. 4, the other embodiment (I) of FIG. 2 and the other embodiment (II) of FIG. 3 may be combined (another embodiment (III)).

Step (e)

In this step, the column bottom liquid (column bottom liquid D) of the third distillation column 5 obtained in the above step (d) is withdrawn from the column bottom part of the third distillation column 5 and supplied to the treatment apparatus 6 via the pipe 61. As a result, the column bottom liquid (column bottom liquid D) is separated into an active component (recovered (meth)acrylic acid ester) and waste oil.

The treatment apparatus 6 may be a distillation column. Here, as a distillation method in the treatment apparatus 6, a known method such as simple distillation (for example, flash distillation) or molecular distillation (thin film distillation) can be used, but the method is not particularly limited thereto. Preferably, the treatment apparatus 6 is a molecular distillation column (thin film distillation column). Alternatively, the treatment apparatus 6 may be heated by a heater such as

a thermosiphon or a forced circulation external heat exchanger. Here, conditions for the distillation are not particularly limited. A distillation pressure is performed under a reduced pressure of, for example, 5 Torr or more and 50 Torr or less (1 Torr = about 1.3 hPa) or under atmospheric pressure, but is not limited thereto. A distillation temperature (particularly, column bottom temperature) is, for example, 80°C or higher and 150°C or lower, preferably 100°C or higher and 130°C or lower, but is not limited thereto. Under such conditions, the active component contained in the column bottom liquid D (hereinafter, also referred to as recovered (meth)acrylic acid ester) and the waste oil can be efficiently separated.

The recovered (meth)acrylic acid ester separated in the treatment apparatus 6 contains the alkene formed by the esterification reaction. Therefore, the recovered (meth)acrylic acid ester may be supplied (recycled) to the reactor 1 via the pipes 64 and 11b to adjust the alkene/secondary alcohol concentration ratio in the reactor 1 (at the time of charging) to 0.001 or more (or the preferable range described above). That is, in an embodiment of the present invention, the alkene-containing product obtained during the production of the (meth)acrylic acid ester is an active component (recovered (meth) acrylic acid ester) obtained after supplying a reaction mixture A obtained in the reaction to at least one distillation column, distilling the reaction mixture A, and supplying a column bottom liquid of the distillation column to a treatment apparatus. In an embodiment of the present invention, the alkene-containing product obtained during the production of the (meth)acrylic acid ester is an active component obtained after subjecting the reaction mixture A obtained in the reaction to the steps (b), (c) and (d) and supplying a column bottom liquid of the third distillation column to a treatment apparatus.

Alternatively, the recovered (meth)acrylic acid ester may be supplied (recycled) to the neutralization and water washing tank 3 (cases of FIGS. 1 and 3) or the reactor 1 (3) (cases of FIGS. 2 and 4) via a pipe 62. The recovered (meth) acrylic acid ester supplied (recycled) to the neutralization and water washing tank 3 is subjected to the same neutralization step and water washing step as in the above step (b), and, if necessary, a distillation step. The gas or condensate liquid after the steps contains the alkene formed by the esterification reaction. Therefore, the gas or condensate liquid may be supplied (recycled) to the reactor 1 to adjust the alkene/secondary alcohol concentration ratio in the reactor 1 (at the time of charging) to 0.001 or more (or the preferable range described above). That is, in an embodiment of the present invention, the alkene-containing product obtained during the production of the (meth)acrylic acid ester is a gas or a condensate liquid of the gas, obtained after supplying a reaction mixture A obtained in the reaction to a distillation column, distilling the reaction mixture A, supplying a column bottom liquid of the distillation column to a treatment apparatus to recover an active component (a gas or a condensate liquid thereof), supplying the active component to a neutralization and water washing tank, and neutralizing and water-washing an oil phase in the neutralization and water washing tank. In an embodiment of the present invention, the alkene-containing product obtained during the production of the (meth)acrylic acid ester is a gas or a condensate liquid of the gas, obtained by subjecting a reaction mixture A obtained in the reaction to the steps (b), (c) and (d), supplying a column bottom liquid of the third distillation column to a treatment apparatus to recover an active component (a gas or a condensate liquid thereof), supplying the oil phase in the neutralization and water washing tank to a neutralization and water washing tank, neutralizing and water-washing the oil phase, and separating it into an aqueous phase and an oil phase, and then distilling the oil phase.

The operation after the second distillation column 4 is continuous, but the recycle (return) of the recovered (meth) acrylic acid ester is also a batch operation because a batch operation is performed in the return destination.

In an embodiment of the present invention, the alkene-containing product obtained during the production of the (meth)acrylic acid ester is at least one selected from the following (i) to (vi):

(i) an oil phase obtained by supplying an azeotropic composition of water formed by the reaction and/or water charged into the reactor and the secondary alcohol to a first distillation column, distilling the azeotropic composition, and condensing a gas distilled off from a column top part of the first distillation column;
(ii) an oil phase obtained by further carrying out the reaction in the presence of an organic solvent (excluding a secondary alcohol), supplying an azeotropic composition of water formed by the reaction and/or water charged into the reactor and the secondary alcohol and/or the organic solvent to a first distillation column, distilling the azeotropic composition, and condensing a gas distilled off from a column top part of the first distillation column;
(iii) a gas or a condensate liquid of the gas, obtained after supplying a reaction mixture A obtained in the reaction to a neutralization and water washing tank, and neutralizing and water-washing the reaction mixture A;
(iv) a gas or a condensate liquid of the gas, distilled off from a column top part of a second distillation column, after supplying a reaction mixture A obtained in the reaction to a neutralization and water washing tank, neutralizing and water-washing the reaction mixture A, then supplying an obtained reaction mixture B to the second distillation column, and distilling the reaction mixture B;
(v) a gas or a condensate liquid of the gas, obtained after supplying a reaction mixture A obtained in the reaction to a distillation column, distilling the reaction mixture A, supplying a column bottom liquid of the distillation column to a treatment apparatus to recover an active component (a gas or a condensate liquid thereof), supplying the active component to a neutralization and water washing tank, and neutralizing and water-washing an oil phase in the neutralization and water washing tank; and
(vi) an active component (recovered (meth)acrylic acid ester) obtained after supplying a reaction mixture A obtained in

the reaction to at least one distillation column, distilling the reaction mixture A, and supplying a column bottom liquid of the distillation column to a treatment apparatus.

The waste oil separated in the treatment apparatus 6 is discarded via a pipe 63.

According to the above method, a (meth)acrylic acid ester can be produced with a high selectivity. In addition, the (meth)acrylic acid ester can be produced with high purity and/or in a high yield. According to the method, a (meth)acrylic acid ester can be produced at low cost.

The embodiments of the present invention have been described in detail, but are illustrative and exemplary and not limitative, and it is apparent that the scope of the present invention should be interpreted based on the appended claims.

The present invention includes the following aspects and forms.

1. A method of producing a (meth)acrylic acid ester, the method including:

    reacting (meth)acrylic acid with a secondary alcohol in a reactor in the presence of an acid-type esterification catalyst and a polymerization inhibitor; and
    supplying an alkene-containing product obtained during production of the (meth)acrylic acid ester to the reactor so that a ratio of an alkene concentration to a secondary alcohol concentration in the reactor is 0.001 or more;

2. The production method according to 1, wherein the alkene concentration in the reactor is 100 ppm by mass or more;
3. The production method according to 1 or 2, wherein the alkene-containing product obtained during the production of the (meth)acrylic acid ester is at least one selected from the following (i) to (iv):

    (i) an oil phase obtained by supplying an azeotropic composition of water formed by the reaction and/or water charged into the reactor and the secondary alcohol to a first distillation column, distilling the azeotropic composition, and condensing a gas distilled off from a column top part of the first distillation column;
    (ii) an oil phase obtained by further carrying out the reaction in the presence of an organic solvent (excluding a secondary alcohol), supplying an azeotropic composition of water formed by the reaction and/or water charged into the reactor and the secondary alcohol and/or the organic solvent to a first distillation column, distilling the azeotropic composition, and condensing a gas distilled off from a column top part of the first distillation column;
    (iii) a gas or a condensate liquid of the gas, obtained after supplying a reaction mixture A obtained in the reaction to a neutralization and water washing tank, and neutralizing and water-washing the reaction mixture A; and
    (iv) a gas or a condensate liquid of the gas, distilled off from a column top part of a second distillation column, after supplying a reaction mixture A obtained in the reaction to a neutralization and water washing tank, neutralizing and water-washing the reaction mixture A, then supplying an obtained reaction mixture B to the second distillation column, and distilling the reaction mixture B;

4. The production method according to any one of 1 to 3, wherein the alkene-containing product obtained during the production of the (meth)acrylic acid ester is a gas or a condensate liquid of the gas, obtained after supplying a reaction mixture A obtained in the reaction to a distillation column, distilling the reaction mixture A, supplying a column bottom liquid of the distillation column to a treatment apparatus to recover an active component (a gas or a condensate liquid thereof), supplying the active component to a neutralization and water washing tank, and neutralizing and water-washing an oil phase in the neutralization and water washing tank;
5. The production method according to any one of 1 to 4, wherein the alkene-containing product obtained during the production of the (meth)acrylic acid ester is an active component (recovered (meth)acrylic acid ester) obtained after supplying a reaction mixture A obtained in the reaction to at least one distillation column, distilling the reaction mixture A, and supplying a column bottom liquid of the distillation column to a treatment apparatus;
6. The production method according to any one of 1 to 5, wherein the secondary alcohol is 2-octanol.

Examples

The effects of the present invention will be described with reference to the following examples and comparative examples. However, the technical scope of the present invention is not limited only to the following examples. In the following examples, operations were performed at room temperature (25 ± 5°C), unless otherwise specified. Unless otherwise specified, "%" and "part(s)" mean "mass%" and "part(s) by mass", respectively.

Comparative Example 1

A glass round-bottomed flask equipped with a stirrer and having a volume of 500 mL was charged with 60.0 g of acrylic acid, 130.1 g of 2-octanol (1.2 mol per mol of acrylic acid), 21.2 g of toluene, 1.2 g of sulfuric acid (2 mass% relative to acrylic acid and 0.6 mass% relative to the total amount of the charged solution) and 0.3 g of phenothiazine and then immersed in an oil bath, and heating was started. In this example, since 2-octene was not added as an alkene, the concentration ratio of 2-octene to 2-octanol was 0, and the 2-octene concentration in the reactor was 0 mass%.

The pressure in the system was gradually reduced from 800 hPa to 180 hPa so that the liquid temperature in the round-bottomed flask was maintained at 110°C.

After a lapse of 8 hours from the time point at which the liquid temperature in the round-bottomed flask reached 110°C as a starting point, the flask was taken out from the oil bath and cooled to terminate the esterification reaction.

When the reaction liquid after completion of the reaction was analyzed according to the following method, 2-octene as an impurity was observed. Therefore, the selectivities of 2-octene and 1-methylheptyl acrylate based on the alcohol (2-octanol) in the reaction liquid after completion of the reaction were calculated according to the following method, and found to be 1.5 mol% and 92.2 mol%, respectively. The conversion rates of acrylic acid and the alcohol (2-octanol) in the reaction liquid after completion of the reaction were calculated according to the following method, and found to be 98.6 mol% and 84.5 mol%, respectively.

Measurement Method of Concentrations of 2-Octanol, 2-Octene, Acrylic Acid, and 1-Methylheptyl (Meth)acrylate

The concentrations of 2-octanol, 2-octene, acrylic acid and 1-methylheptyl (meth)acrylate were measured by quantitative analysis of 2-octanol, 2-octene, acrylic acid and 1-methylheptyl (meth)acrylate in the reaction liquid using gas chromatography (GC). Conditions for the quantitative analysis were as follows.

Gas chromatography: GC-2014 available from Shimadzu Corporation
Column: DB-1 available from Agilent Technologies, Inc. (column length: 30 m × internal diameter: 0.25 mm × film thickness: 0.25 μm
Column temperature: 70°C
Carrier gas: He.

Measurement Method of Concentration of Methacrylic Acid

The concentration of methacrylic acid was measured by quantitative analysis of methacrylic acid in the reaction liquid using gas chromatography (GC). Conditions for the quantitative analysis were as follows.

Gas chromatography: GC-2014 available from Shimadzu Corporation
Column: DB-1701 available from Agilent Technologies, Inc. (column length: 30 m × internal diameter: 0.53 mm × film thickness: 1.0 μm)
Column temperature: 70°C
Carrier gas: He.

Measurement Method of Selectivities of 2-Octene and 1-Methylheptyl (Meth)acrylate

From the results of the quantitative analysis, the selectivities of 2-octene and 1-methylheptyl (meth)acrylate were calculated using the following calculation equations.

[Math. 1]

SELECTIVITY (mol%) OF 2-OCTENE (BASED ON OH)
= [(NUMBER OF MOLES OF 2-OCTENE AFTER REACTION COMPLETION) - (NUMBER OF MOLES OF 2-OCTENE AT REACTION CHARGE)] × 100
/[(NUMBER OF MOLES OF 2-OCTANOL AT REACTION CHARGE) - (NUMBER OF MOLES OF 2-OCTANOL AT REACTION COMPLETION)]

[Math. 2]

$$\text{SELECTIVITY (mol\%) OF 1-METHYLHEPTYL (METH)ACRYLATE (BASED ON OH)} = [(\text{NUMBER OF MOLES OF 1-METHYLHEPTYL (METH)ACRYLATE AFTER REACTION COMPLETION}) - (\text{NUMBER OF MOLES OF 1-METHYLHEPTYL (METH)ACRYLATE AT REACTION CHARGE})] \times 100/[(\text{NUMBER OF MOLES OF 2-OCTANOL AT REACTION CHARGE}) - (\text{NUMBER OF MOLES OF 2-OCTANOL AT REACTION COMPLETION})]$$

Measurement Method of Conversion Rates of (Meth)acrylic Acid and 2-Octanol

The conversion rates of (meth)acrylic acid and 2-octanol were calculated from the results of the same quantitative analysis as described above using the following calculation equations.

[Math. 3]

$$\text{CONVERSION RATE (mol\%) OF (METH)ACRYLIC ACID} = \{1 - [(\text{NUMBER OF MOLES OF (METH)ACRYLIC ACID AFTER REACTION COMPLETION})/(\text{NUMBER OF MOLES OF (METH)ACRYLIC ACID AT REACTION CHARGE})]\} \times 100$$

[Math. 4]

$$\text{CONVERSION RATE (mol\%) OF 2-OCTANOL} = \{1 - [(\text{NUMBER OF MOLES OF 2-OCTANOL AFTER REACTION COMPLETION})/(\text{NUMBER OF MOLES OF 2-OCTANOL AT REACTION CHARGE})]\} \times 100$$

Example 1

The same procedure as in Comparative Example 1 was performed except that 10.0 g of 2-octene was added, the amount of toluene added was changed to 22.4 g, and the amount of sulfuric acid added was changed to 1.3 g, in Comparative Example 1, and that the pressure in the system was gradually reduced from 800 hPa to 200 hPa so that the liquid temperature in the round-bottomed flask was maintained at 110°C. In this example, since the 2-octanol concentration and the 2-octene concentration in the reactor were 58.1 mass% and 4.5 mass%, respectively, the ratio of the 2-octene concentration to the 2-octanol concentration was 0.077.

When the reaction liquid after completion of the reaction was analyzed in the same manner as in Comparative Example 1, formation of 2-octene as an impurity was observed. Therefore, the selectivities of 2-octene and 1-methylheptyl acrylate based on the alcohol (2-octanol) in the reaction liquid after completion of the reaction were calculated according to the same method as in Comparative Example 1, and found to be 1.1 mol% and 92.9 mol%, respectively. Further, the conversion rates of acrylic acid and the alcohol (2-octanol) in the reaction liquid after completion of the reaction were calculated according to the same method as in Comparative Example 1, and found to be 98.2 mol% and 84.1 mol%, respectively.

Example 2

The same procedure as in Comparative Example 1 was performed except that 20.0 g of 2-octene was added, the amount of toluene added was changed to 23.5 g, and the amount of sulfuric acid added was changed to 1.4 g, in Comparative Example 1, and that the pressure in the system was gradually reduced from 800 hPa to 220 hPa so that the liquid temperature in the round-bottomed flask was maintained at 110°C. In this example, since the 2-octanol concentration and the 2-octene concentration in the reactor were 55.3 mass% and 8.5 mass%, respectively, the ratio of the 2-octene concentration to the 2-octanol concentration was 0.154.

When the reaction liquid after completion of the reaction was analyzed in the same manner as in Comparative Example 1, 2-octene as an impurity was observed. Therefore, the selectivities of 2-octene and 1-methylheptyl acrylate based on the alcohol (2-octanol) in the reaction liquid after completion of the reaction were calculated according to the same method as in Comparative Example 1, and found to be 0.7 mol% and 93.3 mol%, respectively. Further, the conversion rates of acrylic acid and the alcohol (2-octanol) in the reaction liquid after completion of the reaction were calculated according to the same method as in Comparative Example 1, and found to be 98.4 mol% and 83.7 mol%,

respectively.

Example 3

The same procedure as in Comparative Example 1 was performed except that 30.0 g of 2-octene was added, the amount of toluene added was changed to 24.6 g, and the amount of sulfuric acid added was changed to 1.4 g, in Comparative Example 1, and that the pressure in the system was gradually reduced from 800 hPa to 240 hPa so that the liquid temperature in the round-bottomed flask was maintained at 110°C. In this example, since the 2-octanol concentration and the 2-octene concentration in the reactor were 52.8 mass% and 12.2 mass%, respectively, the ratio of the 2-octene concentration to the 2-octanol concentration was 0.231.

When the reaction liquid after completion of the reaction was analyzed in the same manner as in Comparative Example 1, 2-octene as an impurity was observed. Therefore, the selectivities of 2-octene and 1-methylheptyl acrylate based on the alcohol (2-octanol) in the reaction liquid after completion of the reaction were calculated according to the same method as in Comparative Example 1, and found to be 0.3 mol% and 93.7 mol%, respectively. Further, the conversion rates of acrylic acid and the alcohol (2-octanol) in the reaction liquid after completion of the reaction were calculated according to the same method as in Comparative Example 1, and found to be 98.5 mol% and 83.2 mol%, respectively.

Example 4

The same procedure as in Comparative Example 1 was performed except that 12.5 g of 2-octene was added, toluene was not added as an azeotropic solvent (amount of toluene added = 0 g), the amount of 2-octanol added was changed to 162.9 g (1.5 mol per mol of acrylic acid), and the amount of sulfuric acid added was changed to 1.4 g, in Comparative Example 1, that the pressure in the system was gradually reduced from 300 hPa to 40 hPa so that the liquid temperature in the round-bottomed flask was maintained at 110°C, and that, after a lapse of 6 hours from the time point at which the liquid temperature in the round-bottomed flask reached 110°C as a starting point, the flask was taken out from the oil bath and cooled to terminate the esterification reaction. In this example, since the 2-octanol concentration and the 2-octene concentration in the reactor were 68.7 mass% and 5.3 mass%, respectively, the ratio of the 2-octene concentration to the 2-octanol concentration was 0.077.

When the reaction liquid after completion of the reaction was analyzed in the same manner as in Comparative Example 1, 2-octene as an impurity was observed. Therefore, the selectivities of 2-octene and 1-methylheptyl acrylate based on the alcohol (2-octanol) in the reaction liquid after completion of the reaction were calculated according to the same method as in Comparative Example 1, and found to be -1.3 mol% and 92.9 mol%, respectively. Further, the conversion rates of acrylic acid and the alcohol (2-octanol) in the reaction liquid after completion of the reaction were calculated according to the same method as in Comparative Example 1, and found to be 93.6 mol% and 57.4 mol%, respectively.

Comparative Example 2

A glass round-bottomed flask equipped with a stirrer and having a volume of 500 mL was charged with 60.0 g of acrylic acid, 116.4 g of 2-octanol (1.1 mol per mol of acrylic acid), 20.2 g of toluene, 4.0 g of a 70 mass% aqueous para-toluenesulfonic acid (PTS) solution (4.7 mass% relative to acrylic acid, 1.4 mass% relative to the total amount of the charged solution (aqueous solution portion not included)), and 0.3 g of phenothiazine and then immersed in an oil bath, and heating was started. In this example, since 2-octene was not added as an alkene, the concentration ratio of 2-octene to 2-octanol was 0, and the 2-octene concentration in the reactor was 0 mass%.

The pressure in the system was gradually reduced from 800 hPa to 190 hPa so that the liquid temperature in the round-bottomed flask was maintained at 110°C.

After a lapse of 9 hours from the time point at which the liquid temperature in the round-bottomed flask reached 110°C as a starting point, the flask was taken out from the oil bath and cooled to terminate the esterification reaction.

When the reaction liquid after completion of the reaction was analyzed in the same manner as in Comparative Example 1, 2-octene as an impurity was observed. Therefore, the selectivities of 2-octene and 1-methylheptyl acrylate based on the alcohol (2-octanol) in the reaction liquid after completion of the reaction were calculated according to the same method as in Comparative Example 1, and found to be 0.5 mol% and 92.6 mol%, respectively. Further, the conversion rates of acrylic acid and the alcohol (2-octanol) in the reaction liquid after completion of the reaction were calculated according to the same method as in Comparative Example 1, and found to be 97.5 mol% and 90.4 mol%, respectively.

Example 5

The same procedure as in Comparative Example 2 was performed except that 5.0 g of 2-octene was added, the amount of 2-octanol added was changed to 119.4 g, the amount of toluene added was changed to 21.0 g, and the amount of the 70 mass% aqueous PTS solution added was changed to 4.2 g, in Comparative Example 2, and that the pressure in the system was gradually reduced from 800 hPa to 200 hPa so that the liquid temperature in the round-bottomed flask was maintained at 110°C. In this example, since the 2-octanol concentration and the 2-octene concentration in the reactor were 56.9 mass% and 2.4 mass%, respectively, the ratio of the 2-octene concentration to the 2-octanol concentration was 0.042.

When the reaction liquid after completion of the reaction was analyzed in the same manner as in Comparative Example 1, 2-octene as an impurity was observed. Therefore, the selectivities of 2-octene and 1-methylheptyl acrylate based on the alcohol (2-octanol) in the reaction liquid after completion of the reaction were calculated according to the same method as in Comparative Example 1, and found to be 0.0 mol% and 93.5 mol%, respectively. Further, the conversion rates of acrylic acid and the alcohol (2-octanol) in the reaction liquid after completion of the reaction were calculated according to the same method as in Comparative Example 1, and found to be 98.8 mol% and 89.4 mol%, respectively.

Example 6

The same procedure as in Comparative Example 2 was performed except that 10.0 g of 2-octene was added, the amount of 2-octanol added was changed to 119.4 g, the amount of toluene added was changed to 21.4 g, and the amount of the 70 mass% aqueous PTS solution added was changed to 4.3 g, in Comparative Example 2, and that the pressure in the system was gradually reduced from 800 hPa to 210 hPa so that the liquid temperature in the round-bottomed flask was maintained at 110°C. In this example, since the 2-octanol concentration and the 2-octene concentration in the reactor were 55.4 mass% and 4.6 mass%, respectively, the ratio of the 2-octene concentration to the 2-octanol concentration was 0.084.

When the reaction liquid after completion of the reaction was analyzed in the same manner as in Comparative Example 1, 2-octene as an impurity was observed. Therefore, the selectivities of 2-octene and 1-methylheptyl acrylate based on the alcohol (2-octanol) in the reaction liquid after completion of the reaction were calculated according to the same method as in Comparative Example 1, and found to be -0.5 mol% and 93.9 mol%, respectively. Further, the conversion rates of acrylic acid and the alcohol (2-octanol) in the reaction liquid after completion of the reaction were calculated according to the same method as in Comparative Example 1, and found to be 98.7 mol% and 88.9 mol%, respectively.

Comparative Example 3

A glass round-bottomed flask equipped with a stirrer and having a volume of 500 mL was charged with 60.0 g of acrylic acid, 119.3 g of 2-octanol (1.1 mol per mol of acrylic acid), 20.0 g of toluene, 2.0 g of a 70 mass% aqueous methanesulfonic acid (MSA) solution (2.3 mass% relative to acrylic acid, 0.7 mass% relative to the total amount of the charged solution (aqueous solution portion not included)), and 0.3 g of phenothiazine and then immersed in an oil bath, and heating was started. In this example, since 2-octene was not added as an alkene, the concentration ratio of 2-octene to 2-octanol was 0, and the 2-octene concentration in the reactor was 0 mass%.

The pressure in the system was gradually reduced from 800 hPa to 190 hPa so that the liquid temperature in the round-bottomed flask was maintained at 110°C.

After a lapse of 9 hours from the time point at which the liquid temperature in the round-bottomed flask reached 110°C as a starting point, the flask was taken out from the oil bath and cooled to terminate the esterification reaction.

When the reaction liquid after completion of the reaction was analyzed in the same manner as in Comparative Example 1, 2-octene as an impurity was observed. Therefore, the selectivities of 2-octene and 1-methylheptyl acrylate based on the alcohol (2-octanol) in the reaction liquid after completion of the reaction were calculated according to the same method as in Comparative Example 1, and found to be 0.3 mol% and 93.1 mol%, respectively. Further, the conversion rates of acrylic acid and the alcohol (2-octanol) in the reaction liquid after completion of the reaction were calculated according to the same method as in Comparative Example 1, and found to be 97.6 mol% and 91.4 mol%, respectively.

Example 7

The same procedure as in Comparative Example 3 was performed except that 5.0 g of 2-octene was added, the amount of 2-octanol added was changed to 119.3 g, the amount of toluene added was changed to 20.7 g, and the amount of

the 70 mass% aqueous MSA solution added was changed to 2.1 g, in Comparative Example 3, and that the pressure in the system was gradually reduced from 800 hPa to 200 hPa so that the liquid temperature in the round-bottomed flask was maintained at 110°C. In this example, since the 2-octanol concentration and the 2-octene concentration in the reactor were 57.5 mass% and 2.4 mass%, respectively, the ratio of the 2-octene concentration to the 2-octanol concentration was 0.042.

When the reaction liquid after completion of the reaction was analyzed in the same manner as in Comparative Example 1, 2-octene as an impurity was observed. Therefore, the selectivities of 2-octene and 1-methylheptyl acrylate based on the alcohol (2-octanol) in the reaction liquid after completion of the reaction were calculated according to the same method as in Comparative Example 1, and found to be -0.2 mol% and 94.1 mol%, respectively. Further, the conversion rates of acrylic acid and the alcohol (2-octanol) in the reaction liquid after completion of the reaction were calculated according to the same method as in Comparative Example 1, and found to be 98.4 mol% and 89.7 mol%, respectively.

Example 8

The same procedure as in Comparative Example 3 was performed except that 10.0 g of 2-octene was added, the amount of 2-octanol added was changed to 119.5 g, the amount of toluene added was changed to 21.3 g, and the amount of the 70 mass% aqueous MSA solution added was changed to 2.2 g, in Comparative Example 3, and that the pressure in the system was gradually reduced from 800 hPa to 210 hPa so that the liquid temperature in the round-bottomed flask was maintained at 110°C. In this example, since the 2-octanol concentration and the 2-octene concentration in the reactor were 56.0 mass% and 4.7 mass%, respectively, the ratio of the 2-octene concentration to the 2-octanol concentration was 0.084.

When the reaction liquid after completion of the reaction was analyzed in the same manner as in Comparative Example 1, 2-octene as an impurity was observed. Therefore, the selectivities of 2-octene and 1-methylheptyl acrylate based on the alcohol (2-octanol) in the reaction liquid after completion of the reaction were calculated according to the same method as in Comparative Example 1, and found to be -0.4 mol% and 94.3 mol%, respectively. Further, the conversion rates of acrylic acid and the alcohol (2-octanol) in the reaction liquid after completion of the reaction were calculated according to the same method as in Comparative Example 1, and found to be 99.1 mol% and 90.3 mol%, respectively.

Comparative Example 4

A glass round-bottomed flask equipped with a stirrer and having a volume of 500 mL was charged with 60.0 g of methacrylic acid, 99.8 g of 2-octanol (1.1 mol per mol of methacrylic acid), 18.2 g of toluene, 3.7 g of a 70 mass% aqueous methanesulfonic acid (MSA) solution (4.3 mass% relative to acrylic acid, 1.4 mass% relative to the total amount of the charged solution), and 0.3 g of phenothiazine and then immersed in an oil bath, and heating was started. In this example, since 2-octene was not added as an alkene, the concentration ratio of 2-octene to 2-octanol was 0, and the 2-octene concentration in the reactor was 0 mass%.

The pressure in the system was gradually reduced from 850 hPa to 320 hPa so that the liquid temperature in the round-bottomed flask was maintained at 120°C.

After a lapse of 9 hours from the time point at which the liquid temperature in the round-bottomed flask reached 120°C as a starting point, the flask was taken out from the oil bath and cooled to terminate the esterification reaction.

When the reaction liquid after completion of the reaction was analyzed in the same manner as in Comparative Example 1, 2-octene as an impurity was observed. Therefore, the selectivities of 2-octene and 1-methylheptyl methacrylate based on the alcohol (2-octanol) in the reaction liquid after completion of the reaction were calculated according to the same method as in Comparative Example 1, and found to be 3.1 mol% and 90.6 mol%, respectively. Further, the conversion rates of methacrylic acid and the alcohol (2-octanol) in the reaction liquid after completion of the reaction were calculated according to the same method as in Comparative Example 1, and found to be 97.8 mol% and 95.7 mol%, respectively.

Example 9

The same procedure as in Comparative Example 4 was performed except that 8.4 g of 2-octene was added, the amount of 2-octanol added was changed to 100.0 g, the amount of toluene added was changed to 19.3 g, and the amount of the 70 mass% aqueous MSA solution added was changed to 3.9 g, in Comparative Example 4, and that the pressure in the system was gradually reduced from 850 hPa to 330 hPa so that the liquid temperature in the round-bottomed flask was maintained at 120°C. In this example, since the 2-octanol concentration and the 2-octene concentration in the reactor were 52.1 mass% and 4.4 mass%, respectively, the ratio of the 2-octene concentration to the 2-octanol concentration was

0.084.

When the reaction liquid after completion of the reaction was analyzed in the same manner as in Comparative Example 1, 2-octene as an impurity was observed. Therefore, the selectivities of 2-octene and 1-methylheptyl methacrylate based on the alcohol (2-octanol) in the reaction liquid after completion of the reaction were calculated according to the same method as in Comparative Example 1, and found to be 1.9 mol% and 93.4 mol%, respectively. Further, the conversion rates of methacrylic acid and the alcohol (2-octanol) in the reaction liquid after completion of the reaction were calculated according to the same method as in Comparative Example 1, and found to be 98.6 mol% and 94.0 mol%, respectively.

Example 10

The same procedure as in Comparative Example 4 was performed except that 15.0 g of 2-octene was added, the amount of 2-octanol added was changed to 100.0 g, the amount of toluene added was changed to 19.8 g, and the amount of the 70 mass% aqueous MSA solution added was changed to 4.0 g, in Comparative Example 4, and that the pressure in the system was gradually reduced from 850 hPa to 340 hPa so that the liquid temperature in the round-bottomed flask was maintained at 120°C. In this example, since the 2-octanol concentration and the 2-octene concentration in the reactor were 50.2 mass% and 7.5 mass%, respectively, the ratio of the 2-octene concentration to the 2-octanol concentration was 0.150.

When the reaction liquid after completion of the reaction was analyzed in the same manner as in Comparative Example 1, 2-octene as an impurity was observed. Therefore, the selectivities of 2-octene and 1-methylheptyl methacrylate based on the alcohol (2-octanol) in the reaction liquid after completion of the reaction were calculated according to the same method as in Comparative Example 1, and found to be 0.9 mol% and 94.4 mol%, respectively. Further, the conversion rates of methacrylic acid and the alcohol (2-octanol) in the reaction liquid after completion of the reaction were calculated according to the same method as in Comparative Example 1, and found to be 98.7 mol% and 93.0 mol%, respectively.

Example 11

The same procedure as in Comparative Example 4 was performed except that 20.0 g of 2-octene was added, the amount of 2-octanol added was changed to 100.0 g, the amount of toluene added was changed to 20.3 g, and the amount of the 70 mass% aqueous MSA solution added was changed to 4.2 g, in Comparative Example 4, and that the pressure in the system was gradually reduced from 850 hPa to 350 hPa so that the liquid temperature in the round-bottomed flask was maintained at 120°C. In this example, since the 2-octanol concentration and the 2-octene concentration in the reactor were 48.8 mass% and 9.8 mass%, respectively, the ratio of the 2-octene concentration to the 2-octanol concentration was 0.200.

When the reaction liquid after completion of the reaction was analyzed in the same manner as in Comparative Example 1, 2-octene as an impurity was observed. Therefore, the selectivities of 2-octene and 1-methylheptyl methacrylate based on the alcohol (2-octanol) in the reaction liquid after completion of the reaction were calculated according to the same method as in Comparative Example 1, and found to be 0.1 mol% and 95.1 mol%, respectively. Further, the conversion rates of methacrylic acid and the alcohol (2-octanol) in the reaction liquid after completion of the reaction were calculated according to the same method as in Comparative Example 1, and found to be 98.6 mol% and 92.2 mol%, respectively.

According to the comparison between Examples 1 to 3 and Comparative Example 1, the comparison between Examples 5 and 6 and Comparative Example 2, the comparison between Examples 7 and 8 and Comparative Example 3, and the comparison between Examples 9 to 11 and Comparative Example 4, it can be seen that the selectivity of 1-methylheptyl acrylate can be significantly improved by adding 2-octene (alkene) to the reactor so that the alkene/secondary alcohol concentration ratio becomes a predetermined ratio. The effect of improving the selectivity from 92.2 mol% (Comparative Example 1) to 92.9 mol% (Example 1) can be said to be a significant difference in effect particularly in the case of mass production of the (meth)acrylic acid ester, and is industrially very beneficial. In addition, in the examples, 2-octene (alkene) was added to the reactor so that the alkene/secondary alcohol concentration ratio became a predetermined ratio at an experimental level, but it is presumed that the same effect can be obtained also when this procedure is applied to the (meth)acrylic acid ester production process as illustrated in FIGS. 1 to 4.

Example 4 is an example in which 2-octanol as a raw material secondary alcohol was used as an azeotropic solvent, but shows the same selectivity as that of Example 1 in which substantially the same operation was carried out except that toluene was used as an azeotropic solvent without increasing the alkene (2-octene).

This application is based on Japanese Patent Application No. 2022-185001 filed on November 18, 2022, the disclosure of which is incorporated herein by reference in its entirety.

Reference Signs List

1: Reactor,
2: First distillation column,
3: Neutralization and water washing tank,
4: Second distillation column,
5: Third distillation column,
6: Treatment apparatus.

## Claims

1. A method of producing a (meth)acrylic acid ester, the method comprising:

   reacting (meth)acrylic acid with a secondary alcohol in a reactor in the presence of an acid-type esterification catalyst and a polymerization inhibitor; and
   supplying an alkene-containing product obtained during production of the (meth)acrylic acid ester to the reactor so that a ratio of an alkene concentration to a secondary alcohol concentration in the reactor is 0.001 or more.

2. The production method according to claim 1, wherein the alkene concentration in the reactor is 100 ppm by mass or more.

3. The production method according to claim 1, wherein the alkene-containing product obtained during the production of the (meth)acrylic acid ester is at least one selected from the following (i) to (iv):

   (i) an oil phase obtained by supplying an azeotropic composition of water formed by the reaction and/or water charged into the reactor and the secondary alcohol to a first distillation column, distilling the azeotropic composition, and condensing a gas distilled off from a column top part of the first distillation column;
   (ii) an oil phase obtained by further carrying out the reaction in the presence of an organic solvent (excluding a secondary alcohol), supplying an azeotropic composition of water formed by the reaction and/or water charged into the reactor and the secondary alcohol and/or the organic solvent to a first distillation column, distilling the azeotropic composition, and condensing a gas distilled off from a column top part of the first distillation column;
   (iii) a gas or a condensate liquid of the gas, obtained after supplying a reaction mixture A obtained in the reaction to a neutralization and water washing tank, and neutralizing and water-washing the reaction mixture A; and
   (iv) a gas or a condensate liquid of the gas, distilled off from a column top part of a second distillation column, after supplying a reaction mixture A obtained in the reaction to a neutralization and water washing tank, neutralizing and water-washing the reaction mixture A, then supplying an obtained reaction mixture B to the second distillation column, and distilling the reaction mixture B.

4. The production method according to claim 1, wherein the alkene-containing product obtained during the production of the (meth)acrylic acid ester is a gas or a condensate liquid of the gas, obtained after supplying a reaction mixture A obtained in the reaction to a distillation column, distilling the reaction mixture A, supplying a column bottom liquid of the distillation column to a treatment apparatus to recover an active component (a gas or a condensate liquid thereof), supplying the active component to a neutralization and water washing tank, and neutralizing and water-washing an oil phase in the neutralization and water washing tank.

5. The production method according to claim 1, wherein the alkene-containing product obtained during the production of the (meth)acrylic acid ester is an active component (recovered (meth)acrylic acid ester) obtained after supplying a reaction mixture A obtained in the reaction to at least one distillation column, distilling the reaction mixture A, and supplying a column bottom liquid of the distillation column to a treatment apparatus.

6. The production method according to claim 1, wherein the secondary alcohol is 2-octanol.

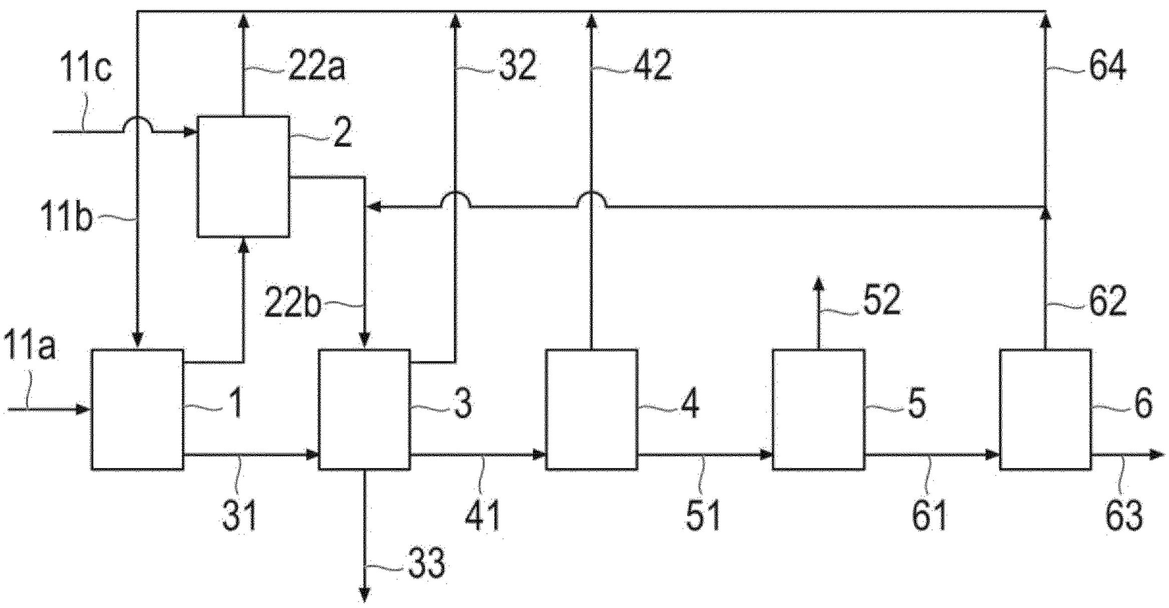


FIG. 1

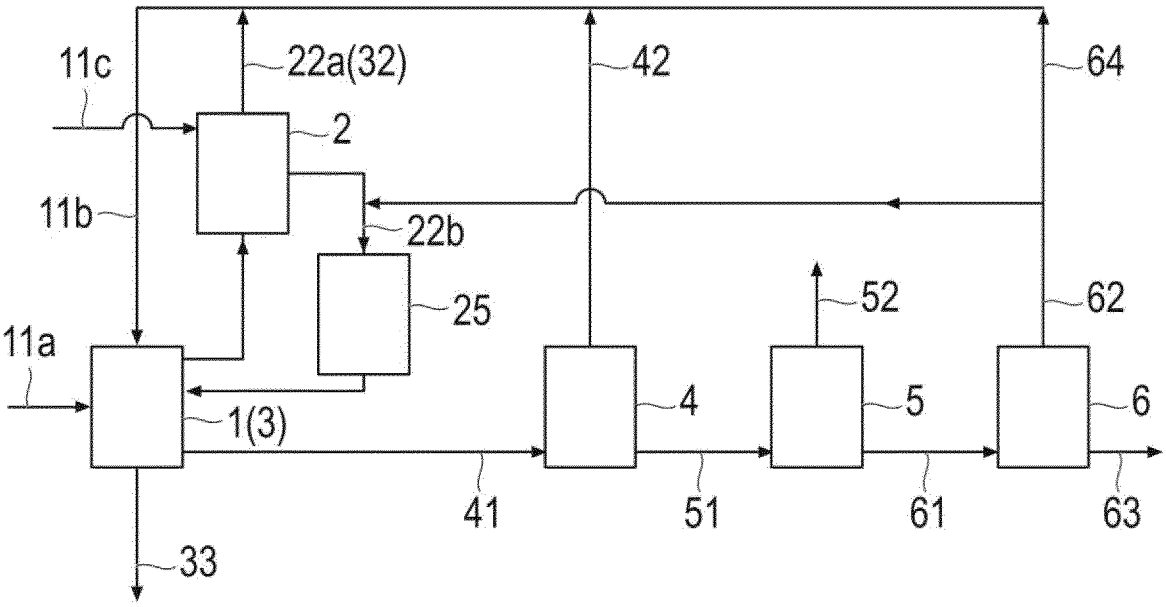


FIG. 2

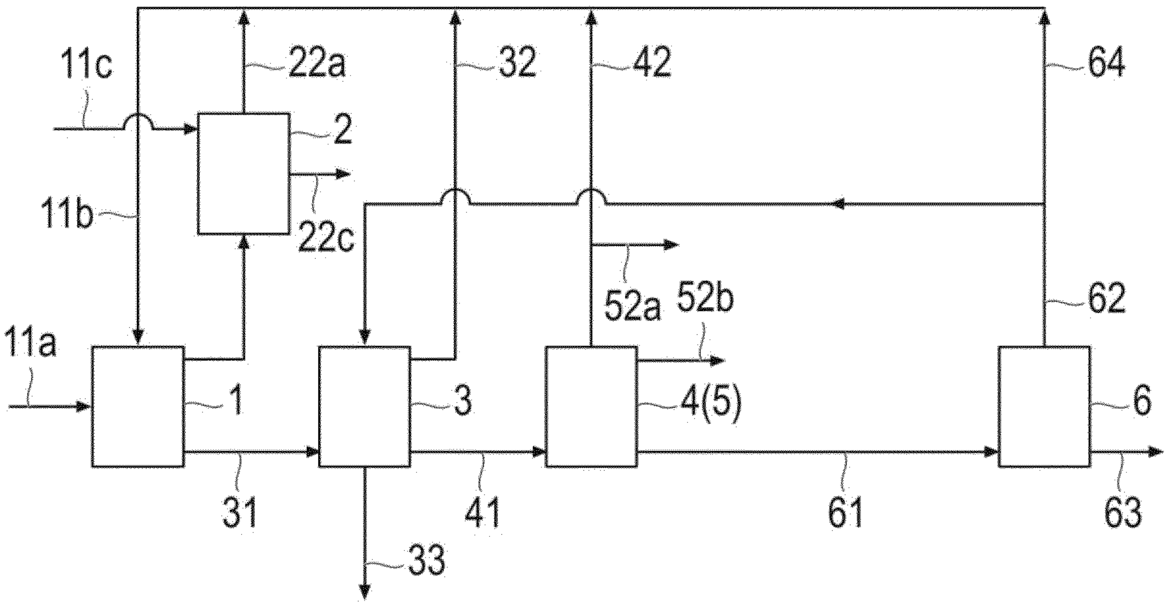


FIG. 3

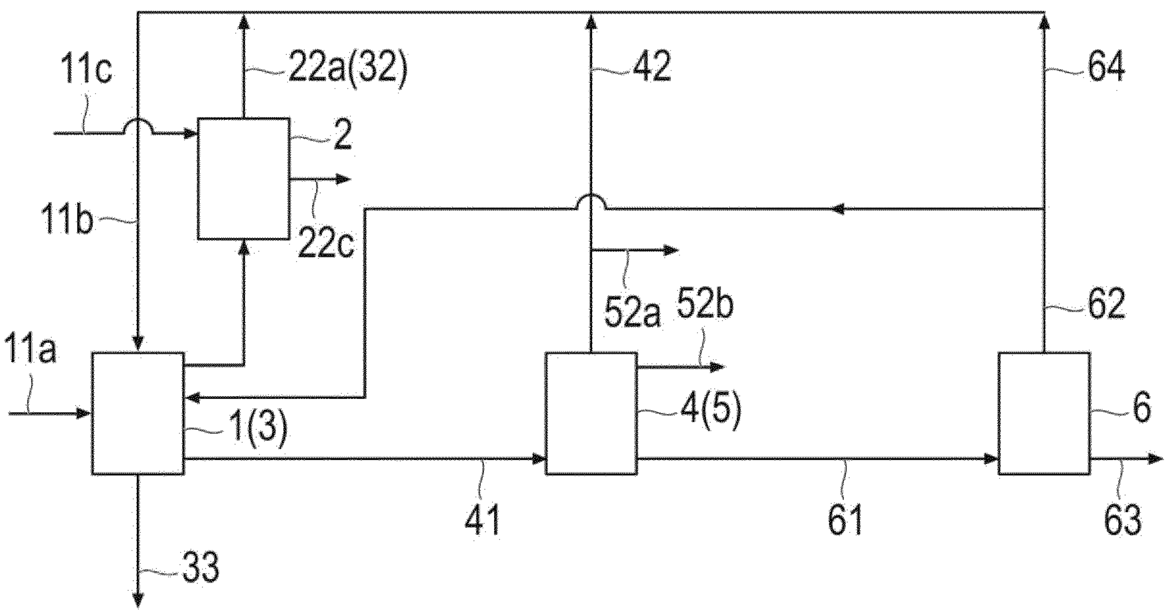


FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6072076 A
- JP 2014534972 T
- US 20150299093 A
- JP 2014 A
- JP 534972 T
- JP 2022185001 A